# EUROPEAN PATENT APPLICATION

(11) **EP 4 755 409 A1**
(43) Date of publication of application: **10.06.2026**
(21) Application number: 24849204.3
(22) Date of filing: 31.07.2024
(51) Int. Cl.: A61L 33/04, A61L 29/08, A61L 29/12, A61L 29/14, A61L 31/12, A61L 31/14

(54) **INSTRUMENT FOR USE IN BLOOD VESSEL, AND COATING AGENT**

(30) Priority: 01.08.2023 JP 2023125315
(71) Applicant: Japan Medical Device Startup Incubation Program, Tokyo, 103-0023 (JP)
(72) Inventor: INUZUKA Naoki, Tokyo 103-0023 (JP); TERAMURA Yuji, Tsukuba-shi, Ibaraki 305-8560 (JP); SATO Yuya, Tsukuba-shi, Ibaraki 305-8560 (JP)
(74) Representative: Appleyard Lees IP LLP
(86) International application number: PCT/JP2024/027320
(87) International publication number: WO 2025/028559

(57) **Abstract**

Provided are an instrument and a coating agent for use in blood vessels, which can be stably supplied, have excellent suppression of clot formation, and are unlikely to inhibit vascular endothelialization. The instrument according to the present invention comprises a base material and a coating layer that covers at least a part of the surface of the base material, and is intended to be used in a blood vessel. The coating layer has a monomolecular layer formed by treating the surface of the base material with a low-molecular-weight compound having a functional group such as an albumin-binding functional group, and satisfies at least one of predetermined requirements.

## Description

### TECHNICAL FIELD

The present invention relates to a device for use inside a blood vessel and to a coating agent.

### BACKGROUND ART

In general, metal base materials for stents and other medical devices for use inside blood vessels have a problem in that they can cause clot formation when placed inside blood vessels for a long time. To address this problem, an indwelling vascular stent has been disclosed that includes a base material and a biocompatible polymer coating on the base material, which aims to prevent clot formation (see, for example, Patent Document 1).

To promote healing after the placement, it is also important to allow the medical device to be quickly covered with vascular endothelial cells (to undergo endothelialization).

### Citation List

### Patent Documents

Patent Document 1: Japanese Patent No. 5463513

### DISCLOSURE OF THE INVENTION

### Problems to be Solved by the Invention

A certain stent includes a base material coated with a biocompatible polymer for preventing clot formation, which includes a structural unit derived from 2-methacryloyloxyethyl phosphorylcholine (MPC). Unfortunately, such a stent has a problem in that it is not easy to undergo endothelialization.

Another medical device includes a base material surface-treated with a silane coupling agent primer and then coated with heparin molecules. Such a medical device has a high ability to resist clot formation and is less likely to inhibit endothelialization. Unfortunately, since it is produced using naturally occurring heparin (e.g., porcine heparin) as a raw material, it has a problem in that its supply can be restricted by social factors.

It is an object of the present invention, which has been made in light of the problems, to provide a device that is for use inside a blood vessel, has a high ability to resist clot formation, is less likely to inhibit endothelialization, and can be easily and stably supplied and to provide a coating agent for such a device.

### Means for Solving the Problems

As a result of diligent studies for solving the problems, the inventors have completed the present invention based on findings that simply surface-treating a base material with a low-molecular-weight compound having such a functional group as an albumin-binding functional group to form a monomolecular layer can provide a solution to the problems. Specifically, the present invention provides the following aspects.

A first aspect of the present invention is directed to a device for use inside a blood vessel, the device including: a base material; and a coating layer at least partially covering the surface of the base material,
the coating layer including a monomolecular layer resulting from surface treatment of the base material with a low-molecular-weight compound having an albumin-binding functional group,
the device satisfying at least one of conditions:
   (1) the coating layer includes the monomolecular layer at its uppermost surface with no polymer layer on the surface of the monomolecular layer,
   (2) the coating layer has an average thickness of 30 nm or less, and
   (3) the coating layer has a surface with at least one of a Si content of 0.5 atm% or more or a P content of 0.5 atm% or more as measured by X-ray photoelectron spectroscopy.

A second aspect of the present invention is directed to a device for use inside a blood vessel, the device including: a base material; and a coating layer at least partially covering the surface of the base material,
the coating layer including a monomolecular layer resulting from surface treatment of the base material with a low-molecular-weight compound having at least one functional group selected from the group consisting of an amino group, a halogen group, a carboxy group, and an alkyl group,
the device satisfying at least one of conditions:
   (1) the coating layer includes the monomolecular layer at its uppermost surface with no polymer layer on the surface of the monomolecular layer,
   (2) the coating layer has an average thickness of 30 nm or less, and
   (3) the coating layer has a surface with at least one of a Si content of 0.5 atm% or more or a P content of 0.5 atm% or more as measured by X-ray photoelectron spectroscopy.

A third aspect of the present invention is directed to a coating agent for use in manufacture of the device according to the first aspect, the coating agent including a low-molecular-weight compound having an albumin-binding functional group.

A fourth aspect of the present invention is directed to a coating agent for use in manufacture of the device according to the second aspect, the coating agent including a low-molecular-weight compound having at least one functional group selected from the group consisting of an amino group, a halogen group, a carboxy group, and an alkyl group.

### Effects of the Invention

The present invention provides a device that is for use inside a blood vessel, has a high ability to resist clot formation, is less likely to inhibit endothelialization, and can be easily and stably supplied and provides a coating agent for such a device.

### BRIEF DESCRIPTION OF THE DRAWINGS

FIG. 1 is a graph showing the results of a clot formation test for examples;
FIG. 2 is a graph showing the results of a clot formation test for examples;
FIG. 3 is a graph showing the results of a clot formation test for examples;
FIG. 4 is an SEM image showing the result of a clot formation test for an example;
FIG. 5 is an SEM image showing the result of a clot formation test for an example;
FIG. 6 is an SEM image showing the result of a clot formation test for an example;
FIG. 7 is an SEM image showing the result of a clot formation test for an example;
FIG. 8 is an SEM image showing the result of a clot formation test for an example;
FIG. 9 is an SEM image showing the result of a clot formation test for an example;
FIG. 10 is an SEM image showing the result of a clot formation test for an example;
FIG. 11 is an SEM image showing the result of a clot formation test for an example;
FIG. 12 is a graph showing the results of coagulation activity evaluation for examples;
FIG. 13 is a graph showing the results of an endothelialization test for examples;
FIG. 14 shows images showing the results of an endothelialization test for examples; and
FIG. 15 is a graph showing the results of a test for endothelialization in animals with respect to examples.

### PREFERRED MODE FOR CARRYING OUT THE INVENTION

Hereinafter, embodiments of the present invention will be described in detail. It will be understood that the embodiments are not intended to limit the present invention and may be appropriately modified for implementation without departing from the gist of the present invention.

### Device

The present invention is directed to a device including: a base material; and a coating layer at least partially covering the surface of the base material. The coating layer includes a monomolecular layer resulting from surface treatment of the base material with a low-molecular-weight compound having an albumin-binding functional group. The device satisfies at least one of the following conditions:
(1) the coating layer includes the monomolecular layer at its uppermost surface with no polymer layer on the surface of the monomolecular layer,
(2) the coating layer has an average thickness of 30 nm or less, and
(3) the coating layer has a surface with at least one of a Si content of 0.5 atm% or more or a P content of 0.5 atm% or more as measured by X-ray photoelectron spectroscopy.

As mentioned above, the inventors have found that a device having a high ability to resist clot formation and being less likely to inhibit endothelialization can be obtained by simply surface-treating a base material with a low-molecular-weight compound having an albumin-binding functional group to form a monomolecular layer without forming any polymer layer on the surface of the monomolecular layer. The conditions (1), (2), and (3) represent such features of the present invention.

The reasons for such advantageous effects remain to be clarified. It may be speculated that the coating layer has albumin-binding functional groups exposed at its uppermost surface, to which blood albumin can adsorb to cover the surface of the device in advance, so that the surface of the device can be less prone to adsorption of coagulation system proteins, can resist clot formation, and can be less likely to inhibit endothelialization.

For condition (2), the coating layer has an average thickness of 30 nm or less, preferably 20 nm or less, more preferably 10 nm or less, even more preferably 5 nm or less. The coating layer with an average thickness in such a range means that it includes no polymer layer or only part of it may include a polymer layer. The average thickness of the coating layer may have any lower limit. For example, the average thickness of the coating layer may have a lower limit of 0.1 nm or more.

In the specification, the average thickness of the coating layer refers to the value determined by a process including: scraping off, in water, the coating layer at three positions using the cantilever in an atomic force microscope (AFM); determining the thickness of the coating layer at each position from the level difference between the surface of the coating layer and the exposed surface of the base material; and arithmetically averaging the thicknesses of the coating layer at the three positions.

For condition (3), the coating layer has a surface with at least one of a Si content of 0.5 atm% or more or a P content of 0.5 atm% or more, preferably with at least one of a Si content of 0.8 atm% or more or a P content of 0.8 atm% or more, as measured by X-ray photoelectron spectroscopy. The coating layer having a surface with a Si or P content in such a range means that most of the monomolecular layer including a low-molecular-weight compound having a Si or P atom is located on the surface side of the coating layer. The Si or P content may have any upper limit. For example, the Si content and the P content may each have an upper limit of 15 atm% or less, 10 atm% or less, or 5 atm% or less. In the specification, the Si or P content refers to the value determined by a process including: measuring the surface of the coating layer by X-ray photoelectron spectroscopy to determine the peak area for Si or P; and calculating the atomic content of Si or P from the peak area.

### Base Material

The base material may be any type. The base material may be one conventionally known for use in devices used inside blood vessels.

The base material preferably includes a highly biocompatible material with high stiffness. Examples of such a material include titanium, nickel, stainless steel, platinum, gold, silver, copper, iron, chromium, cobalt, aluminum, molybdenum, manganese, tantalum, tungsten, niobium, magnesium, calcium, and alloys containing any of those metals. Alternatively, the base material may include synthetic resin, such as polyolefin (e.g., polyethylene (PE), polypropylene (PP)), polyamide, polyvinyl chloride, polyphenylene sulfide, polycarbonate, polyether, or polymethyl methacrylate. Alternatively, the base material may include biodegradable resin (biodegradable polymer), such as polylactic acid (PLA), polyhydroxybutyrate (PHB), polyglycolic acid (PGA), or poly(ε-caprolactone). In particular, the base material preferably includes titanium, nickel, stainless steel, platinum, gold, silver, copper, magnesium, or an alloy containing any of those metals. Examples of the alloy include Ni-Ti alloys, Cu-Mn alloys, Cu-Cd alloys, Co-Cr alloys, Cu-Al-Mn alloys, Au-Cd-Ag alloys, Ti-Al-V alloys, and alloys of magnesium with Zr, Y, Ti, Ta, Nd, Nb, Zn, Ca, Al, Li, or Mn. Alternatively, the base material may include non-biodegradable resin. As mentioned above, the base material may include any biocompatible material.

The base material may have any suitable shape appropriately selected depending on the type of the device. For example, the device may be a stent. In such a case, the base material may have a common stent structure with a radially expandable and contractible framework.

The base material preferably has a linear portion. The base material with a linear portion can have a relatively large surface area and be relatively prone to clot formation. Thus, the present invention is advantageous for the base material with a linear portion.

### Coating Layer

The coating layer at least partially covers the surface of the base material and includes a monomolecular layer.

The coating layer may cover the entire surface of the base material.

### Monomolecular Layer

As used herein, the term "monomolecular layer" refers to a layer that includes organic molecules, has a thickness substantially equal to the length of one molecule of the component, and is physically or chemically bonded to the base material. For example, the monomolecular layer may be a self-assembled monomolecular layer (SAM). The monomolecular layer includes molecules physically or chemically bonded to the base material. To such molecules, 1 to 10 molecules (preferably 1 to 5 molecules, more preferably 1 to 3 molecules) of the same component may be bonded like a chain or network.

The monomolecular layer is a product resulting from surface treatment of the base material with a low-molecular-weight compound having an albumin-binding functional group.

For example, the monomolecular layer has a thickness of 0.1 to 5 nm or 0.1 to 3 nm.

In the specification, the thickness of the monomolecular layer refers to the value measured by optical ellipsometry.

### Low-Molecular-Weight Compound Having Albumin-Binding Functional Group

The term "albumin-binding functional group" refers to a functional group that adsorbs to and physically or chemically binds to blood albumin.

The low-molecular-weight compound having an albumin-binding functional group may be a low-molecular-weight compound having at least one functional group selected from the group consisting of an amino group, a halogen group, a carboxy group, and an alkyl group (hereinafter also simply referred to as the "low-molecular-weight compound"). In particular, against clotting activity or for antibacterial activity (specifically, but not limited to, antibacterial activity against Gram-negative bacteria), the low-molecular-weight compound preferably has an amino group.

The alkyl group is preferably a linear or branched alkyl group having 1 to 20 carbon atoms, more preferably a linear alkyl group having 1 to 5 carbon atoms.

The low-molecular-weight compound having an albumin-binding functional group preferably has an additional functional group capable of covalently binding to the surface of the base material. This means that in the monomolecular layer, the low-molecular-weight compound having an albumin-binding functional group preferably has a structural unit covalently bonded to the surface of the base material.

The functional group capable of covalently binding to the surface of the base material may be any suitable group appropriately selected depending on the type of the base material. Examples of such a functional group include an alkoxy group, a thiol group, and a phosphonate group (-PO₃H₂).

The low-molecular-weight compound having an albumin-binding functional group is preferably a silane coupling agent, a thiol compound, or a phosphonate compound, more preferably a silane coupling agent, which can easily and stably bind to the surface of the base material.

Examples of the silane coupling agent include amino group-containing silane coupling agents, such as (3-aminopropyl)triethoxysilane (APTES) and (3-aminopropyl)trimethoxysilane; and alkyl group-containing silane coupling agents, such as trimethoxymethylsilane (TMMS) and triethoxymethylsilane. In particular, against clotting activity or for antibacterial activity (specifically, but not limited to, antibacterial activity against Gram-negative bacteria), the silane coupling agent is preferably an amino group-containing silane coupling agent.

The thiol compound is a compound having a thiol group, examples of which include 3-mercaptopropionic acid (MPA), thioglycolic acid, 4-mercaptobutanoic acid, 5-mercaptopentanoic acid, 6-mercaptohexanoic acid, 7-mercaptoheptanoic acid, 8-mercaptooctanoic acid, 9-mercaptononanoic acid, 10-mercaptodecanoic acid, 11-mercaptoundecanoic acid, 12-mercaptododecanoic acid, and other carboxy group-containing thiol compounds.

The phosphonate compound is a compound having a phosphonate group, examples of which include alkyl group-containing phosphonate compounds, such as octadecylphosphonic acid.

The low-molecular-weight compound having an albumin-binding functional group preferably has a molecular weight of 1,000 or less, more preferably 500 or less.

### Polymer Layer

The coating layer may further include a polymer layer. Preferably, the coating layer includes no polymer layer. In the absence of a polymer layer, nothing can hinder the exposure of the albumin-binding functional group from the monomolecular layer at the uppermost surface of the coating layer, which will lead to easy achievement of the advantageous effects of the present invention.

In the specification, the term "polymer layer" refers to a layer including a polymer that does not constitute the monomolecular layer. For example, the polymer layer may have a thickness of 1 nm or more, 10 nm or more, or 10 nm to 10 µm.

The polymer layer may include any type of polymer, such as a polymer generally used to provide, for medical devices, the ability to resist clot formation. For example, the polymer layer may include a polymer having a structural unit derived from 2-methacryloyloxyethyl phosphorylcholine (MPC) or a polymer including a heparin molecule.

### Method for Manufacturing the Device

For example, a non-limiting method for manufacturing the device may include surface-treating the base material with the low-molecular-weight compound having an albumin-binding functional group to form the monomolecular layer.

The surface treatment to form the monomolecular layer may be performed using any suitable method, such as a conventionally known method for forming a self-assembled monomolecular layer. For example, such a method may include immersing the base material in a solution containing the low-molecular-weight compound having an albumin-binding functional group; and then drying the solution on the base material.

### Applications

The device for use inside a blood vessel is preferably to be placed partially or entirely inside a blood vessel. The device is preferably a medical device, more preferably a therapeutic implant for use inside a blood vessel.

The therapeutic implant for use inside a blood vessel may be a stent, a coil, a shunt, or a catheter. In particular, the device is preferably a stent.

Besides the therapeutic medical device, the device may be a device that is to be placed inside the body (blood vessel) of an animal, such as human, for connection to an external network.

### Another Mode of the Device

In another mode of the device, the monomolecular layer includes a low-molecular-weight compound having at least one functional group selected from the group consisting of an amino group, a halogen group, a carboxy group, and an alkyl group.

Such a low-molecular-weight compound having at least one functional group selected from the group consisting of an amino group, a halogen group, a carboxy group, and an alkyl group may be the same as that described above in the section "Low-Molecular-Weight Compound Having Albumin-Binding Functional Group".

### Coating Agent

The coating agent is for use in manufacture of the device and includes a low-molecular-weight compound having an albumin-binding functional group.

The low-molecular-weight compound having an albumin-binding functional group in the coating agent may be the same as that described above in the section "Low-Molecular-Weight Compound Having Albumin-Binding Functional Group".

The coating agent may further include an additional component, such as an organic solvent, in addition to the low-molecular-weight compound having an albumin-binding functional group. The coating agent may contain any amount of the low-molecular-weight compound having an albumin-binding functional group.

### Another Mode of the Coating Agent

Another mode of the coating agent may include a low-molecular-weight compound having at least one functional group selected from the group consisting of an amino group, a halogen group, a carboxy group, and an alkyl group.

Such a low-molecular-weight compound having at least one functional group selected from the group consisting of an amino group, a halogen group, a carboxy group, and an alkyl group may be the same as that described above in the section "Low-Molecular-Weight Compound Having Albumin-Binding Functional Group".

### EXAMPLES

Hereinafter, the present invention will be specifically described with reference to examples, which are not intended to limit the present invention.

### Example 1

The surface of a commercially available stent base material (Ni-Ti alloy) was hydrophilized by plasma irradiation. The treated stent was immersed in a 5 mass% solution of (3-aminopropyl)triethoxysilane (APTES) at 20°C for 120 minutes. The treated stent was taken out of the solution and dried at 70°C.

The resulting stent had a coating layer with an average thickness of at most 3 nm, which had no polymer layer. The surface of the coating layer had a Si content of 1 atm% as measured by X-ray photoelectron spectroscopy.

Instead of the commercially available stent base material, a metal sheet (Ni-Ti alloy sheet) was also subjected to the same process to form a treated metal sheet. A treated metal sheet was also prepared in the same manner in each of Comparative Examples 1 to 4 below.

### Comparative Example 1

The surface of a commercially available stent base material (Ni-Ti alloy) was hydrophilized by plasma irradiation. The treated stent was immersed in a 2 mass% solution of 2-(methacryloyloxy)ethyl phosphorylcholine (MPC) for 10 minutes. The treated stent was taken out of the solution and dried at 70°C.

The resulting stent had a coating layer including no monomolecular layer.

### Comparative Example 2

The surface of a commercially available stent base material (Ni-Ti alloy) was hydrophilized by plasma irradiation. The treated stent was immersed in a 4 mass% solution of 2-(methacryloyloxy)ethyl phosphorylcholine (MPC) for 10 minutes. The treated stent was taken out of the solution and dried at 70°C.

The resulting stent had a coating layer including no monomolecular layer.

### Comparative Example 3

The surface of a commercially available stent base material (Ni-Ti alloy) was hydrophilized by plasma irradiation. The treated stent was immersed in a 5 mass% solution of 3-aminopropyltriethoxysilane (APTES) for 120 minutes. The treated stent was taken out of the solution and vacuum-dried at 70°C.

Next, the dried stent was immersed overnight in a 0.1 to 1.0 mass% solution of heparin. The treated stent was taken out of the solution and dried.

The resulting stent had a coating layer including a polymer layer including heparin molecules. The coating layer had an average thickness of 100 nm. The surface of the coating layer had a Si content of less than 0.5 atm% as measured by X-ray photoelectron spectroscopy.

### Comparative Example 4

A commercially available stent base material (Ni-Ti alloy) was used as is.

### Example 2

The surface of a commercially available stent base material (Ni-Ti alloy) was hydrophilized by plasma irradiation. The treated stent was immersed in a 5 mass% solution of trimethoxymethylsilane (TMMS) at 20°C for 120 minutes. The treated stent was taken out of the solution and vacuum-dried at 70°C.

The resulting stent had a coating layer including no polymer layer. The coating layer had an average thickness of at most 3 nm. The surface of the coating layer had a Si content of 1 atm% as measured by X-ray photoelectron spectroscopy.

### Example 3

The surface of a commercially available stent base material (Ni-Ti alloy) was hydrophilized by plasma irradiation. The treated stent was immersed in a 10 mass% solution of 3-mercaptopropionic acid (MPA) at 20°C for 120 minutes. The treated stent was taken out of the solution and vacuum-dried at 70°C.

The resulting stent had a coating layer including no polymer layer. The coating layer had an average thickness of at most 3 nm.

### Comparative Example 5

The surface of a commercially available stent base material (Ni-Ti alloy) was hydrophilized by plasma irradiation. The treated stent was immersed in a 10 mass% solution of 3-mercapto-1-propanol (MPOH), which had no albumin-binding functional group, for 120 minutes. The treated stent was taken out of the solution and vacuum-dried at 70°C.

### Clot Formation Test

The stent of each of Example 1 and Comparative Examples 1 to 4 was evaluated for the ability to resist clot formation using the Chandler loop model. The test was performed three times on each stent, and the results were averaged for evaluation. Specifically, the test was performed as described below.

The stent was placed in a tube with an inner diameter of 4.0 mm and a length of 600 mm. Seven mL of blood was collected from a healthy donor into a blood collection tube. After 70 µL of a heparin solution (a solution prepared by adding heparin at 100 IU/mL to physiological saline) was added to the blood, the mixture was agitated by gently moving the blood collection tube up and down. One mL of the blood mixture was dispensed and immediately subjected to the counting of blood cells. Two mL of the remaining blood mixture was added to the tube containing the stent, and the loop was closed. The tube was mounted on a rotator and spun at 37°C and 10 rpm for 3 hours.

The stent of each of Examples 2 and 3 and Comparative Example 5 was also evaluated for the ability to resist clot formation in the same manner except that blood collected from a different healthy donor was used.

### Blood Cell Counting

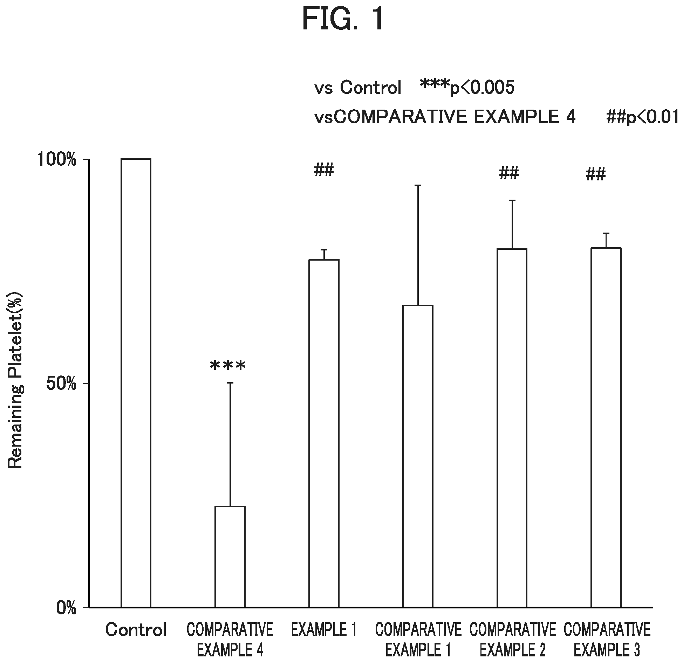

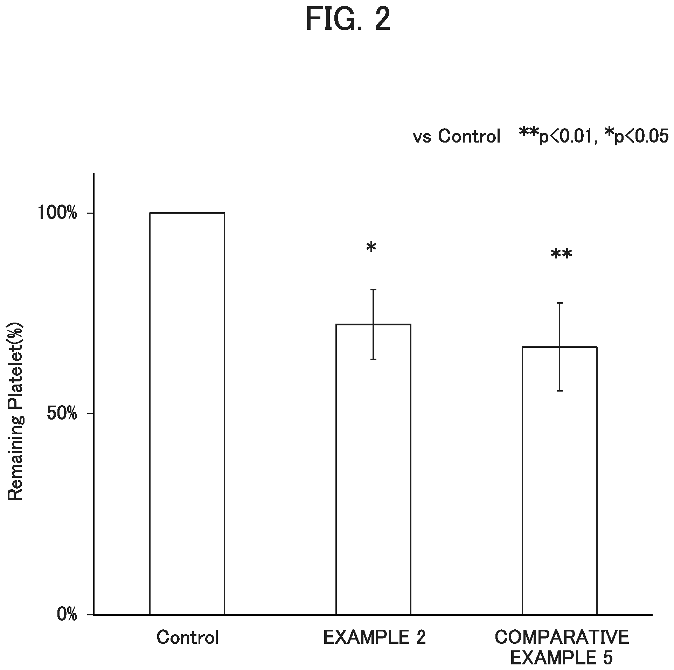

EDTA was added to each blood sample recovered from the tube before the counting of blood cells. Immediately after the collection of blood, the number of blood platelets was counted as a control. The ratio (%) of the number of blood platelets for each of Example 1 and Comparative Examples 1 to 4 to the control was calculated and shown in FIG. 1. The ratio (%) of the number of blood platelets for each of Example 2 and Comparative Example 5 to the control was also calculated and shown in FIG. 2. A higher platelet count indicates a lower level of coagulation reaction and a higher ability to resist clot formation.

### Appearance Evaluation

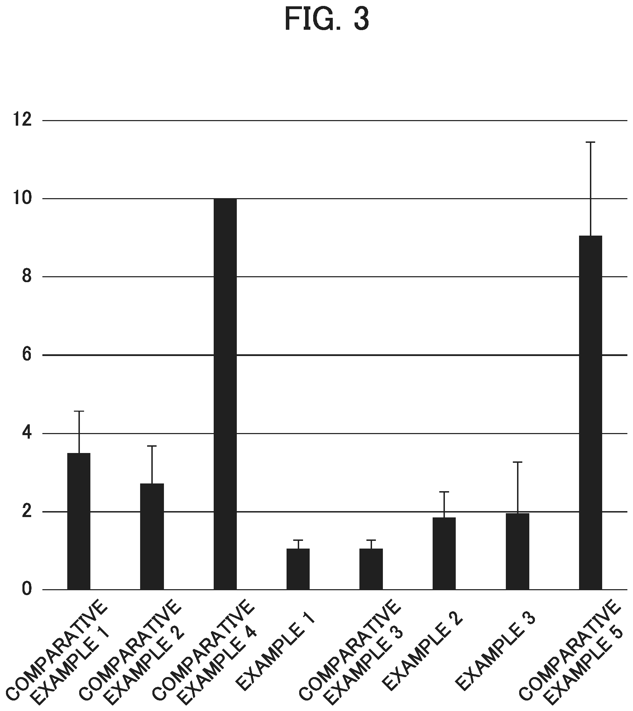

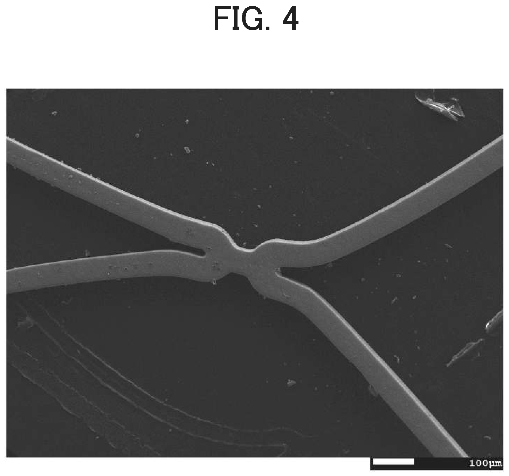

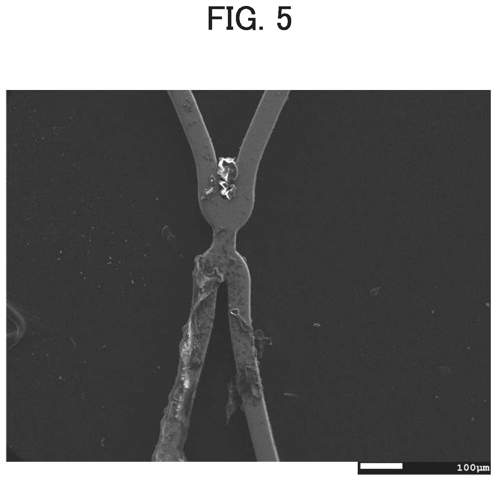

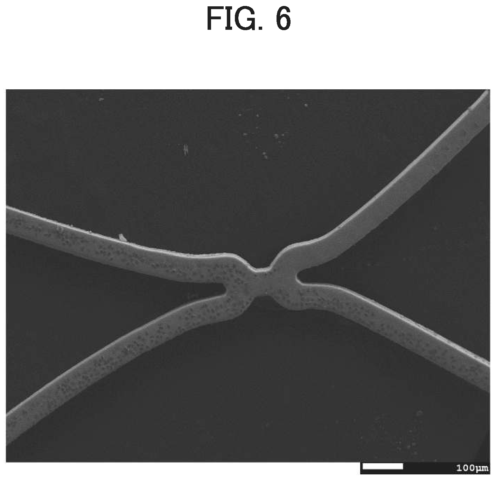

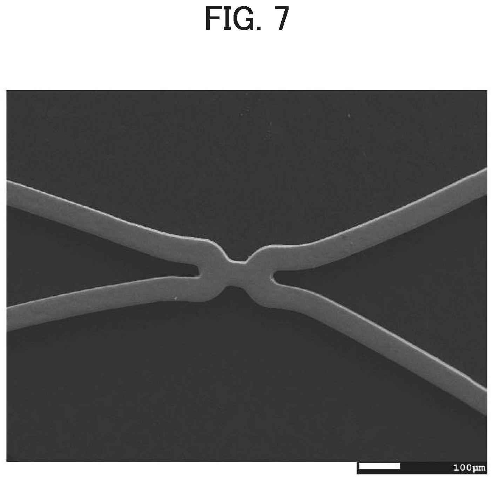

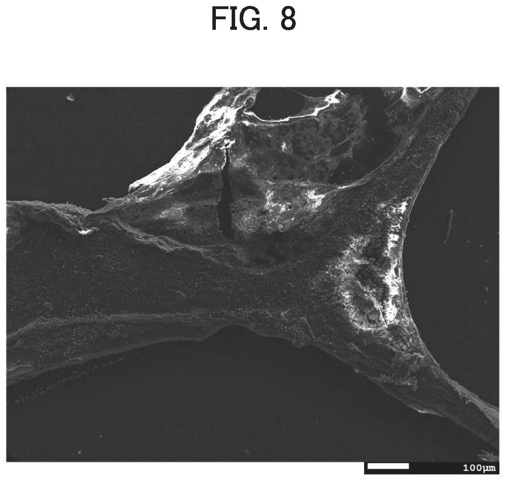

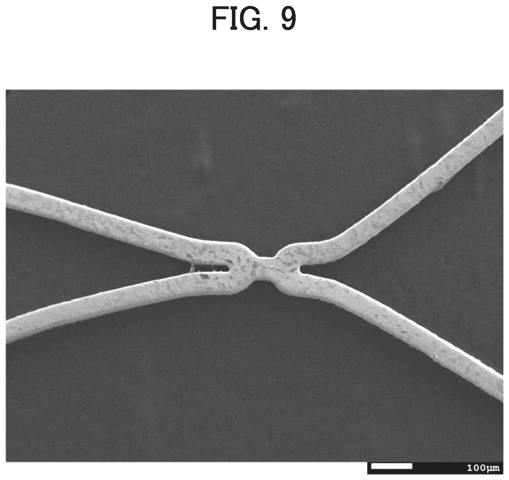

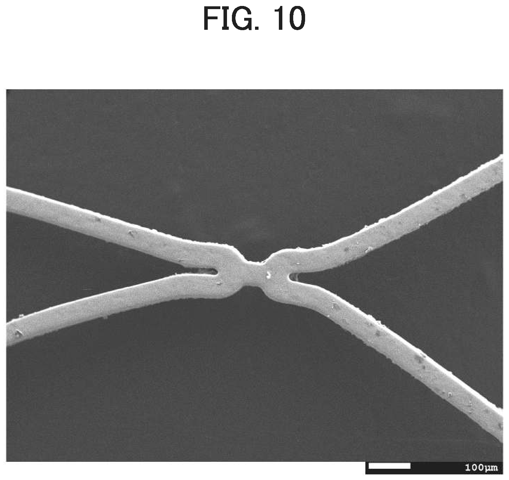

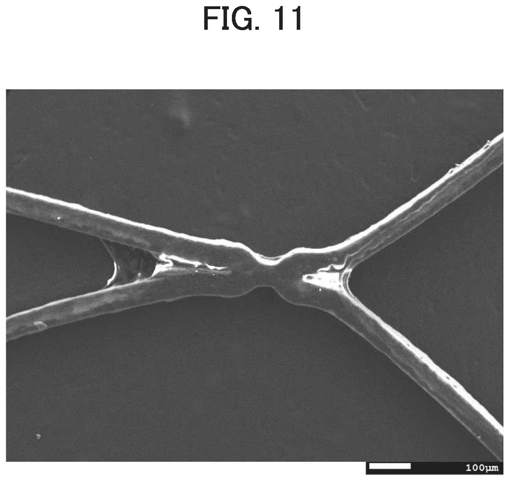

The degree of clot formation on each stent retrieved from the tube was observed using SEM and evaluated according to the criteria below. The results for Examples 1 to 3 and Comparative Examples 1 to 5 are shown in FIG. 3. The SEM images for Example 1 and Comparative Examples 1, 2, 3, and 4 are shown in FIGS. 4, 5, 6, 7, and 8, respectively, and the SEM images for Examples 2 and 3 and Comparative Example 5 are shown in FIGS. 9, 10, and 11, respectively.
1: Almost no blood cells adhere to the stent.
2: Platelets with their shape intact adhere to part of the stent.
3: Platelets with their shape intact adhere over the stent.
4: Blood clots and platelets adhere to part of the stent.
5: Blood clots adhere to part of the stent, and platelets adhere over the stent.
6: A certain amount of blood clots adhere to the stent, and platelets adhere over the stent.
7: Blood clots and platelets adhere over the stent.
8: Struts of a portion of the stent are completely covered with blood cells.
9: Struts of two or more portions of the stent are completely covered with blood cells.
10: All struts are completely covered with blood cells.

### Coagulation Activity Evaluation

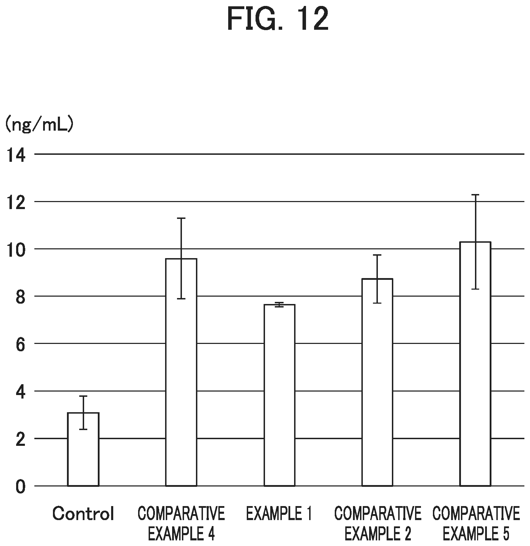

Each blood sample recovered from the tube was centrifuged at 4°C and 2,500 rpm, and the supernatant plasma was collected. The TAT (thrombin-antithrombin complex) in the collected plasma was measured for concentration (ng/mL). The results for Example 1 and Comparative Examples 2, 4, and 5 are shown in FIG. 12. The control indicates the TAT concentration prior to the test. A lower TAT concentration indicates lower coagulation activity and a higher ability to resist clot formation.

### Endothelialization Test

Each of the metal sheets was evaluated for the degree of promotion of endothelialization. Specifically, the test was performed as described below.

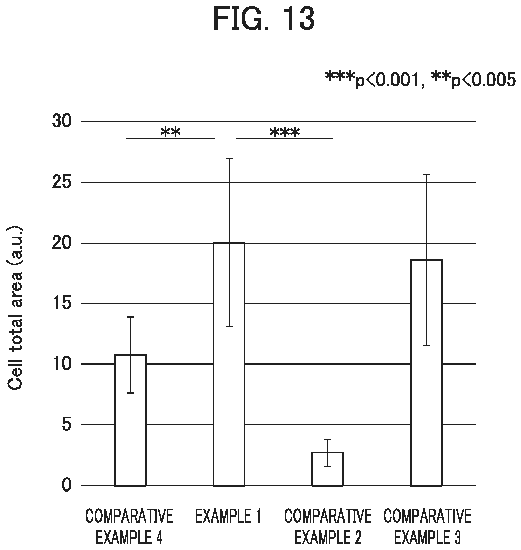

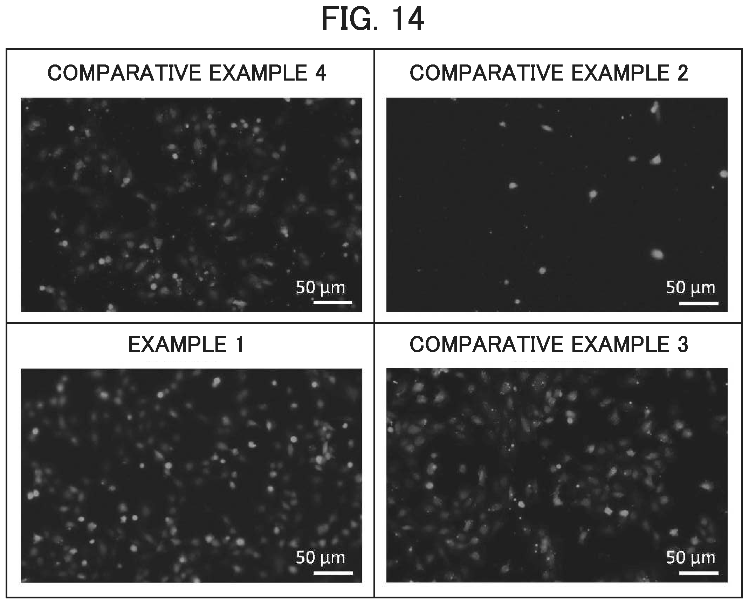

Each of the metal sheets of Example 1 and Comparative Examples 2 to 4 was placed on a cell culture dish, and vascular endothelial cells were seeded on the metal plate surface. Twenty-four hours after the seeding, the cells were observed and imaged using a fluorescence microscope. The area of each fluorescent region was measured, and the total cell adhesion area was calculated. The results are shown in FIG. 13. The images for Example 1 and Comparative Examples 2 to 4 are each shown in FIG. 14. The result obtained for each of Examples 2 and 3 was similar to that for Example 1.

### Test for Endothelialization in Animals

The stent of Example 1 and the stent of Comparative Example 1 were placed respectively in the right and left internal mammary arteries of each of two minipigs (27 kg male Göttingen minipigs), left for 7 days, and then evaluated for the degree of endothelialization thereon. Specifically, the test was performed as described below.

### Stent Placement

A guiding catheter was delivered from the minipig's femoral artery to the right internal mammary artery through a sheath introducer. The diameter of the right internal mammary artery was measured and confirmed to fall within the adequate range for placement of the stent. A microcatheter was delivered to the right internal mammary artery through the guiding catheter. The stent of Example 1 was delivered to the right internal mammary artery through the microcatheter and left for 7 days.

At the same time, like the stent of Example 1, the stent of Comparative Example 1 was placed in the left internal mammary artery of the same minipig and left for 7 days.

Similarly, the stent of Example 1 and the stent of Comparative Example 1 were placed respectively in the right and left internal mammary arteries of the other minipig and left for 7 days.

### Harvest and Examination

A guiding catheter was delivered from the minipig's femoral artery to the internal mammary artery through a sheath introducer. A vascular imaging agent was administered through the guiding catheter, and angiography was performed to confirm that the blood flow and the blood vessel were normal. After the minipig was euthanized, the internal mammary artery was harvested. The harvested internal mammary artery was fixed by immersion in formalin.

After the fixed internal mammary artery was embedded in resin, hematoxylin-eosin-stained (HE stained) sections were prepared from the embedded sample of the internal mammary artery portion containing the stent. Eight sections were prepared per internal mammary artery (16 sections in total per example).

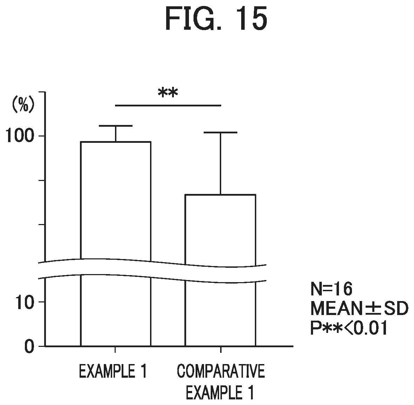

The tissue coverage rate for each section was calculated from the equation below, and the average and standard deviation of the tissue coverage rates were calculated for each of the example and the comparative example. The results are shown in FIG. 15.

Tissue coverage rate (%) = (the number of struts covered with endothelial-like cells or neointimal tissues/the total number of struts) x 100

The results of the clot formation test and the endothelialization test show that the stents of Comparative Examples 1 and 2, which have no monomolecular layer derived from a low-molecular-weight compound having an albumin-binding functional group and include a base material coated with a polymer including an MPC-derived structural unit, inhibit endothelialization. The results also show that the stent of Comparative Example 5, which has a monomolecular layer derived from a low-molecular-weight compound having no albumin-binding functional group, has a poor ability to resist clot formation.

On the other hand, the results show that the stents of Examples 1 to 3, which have a monomolecular layer derived from a low-molecular-weight compound having an albumin-binding functional group, have a high ability to resist clot formation like the stent of Comparative Example 3, which has a coating of heparin molecules, and are less likely to inhibit endothelialization while they are inexpensive and will not suffer from supply issues since they do not include any material produced using naturally occurring heparin as a raw material.

## Claims

1. A device for use inside a blood vessel, the device comprising: a base material; and a coating layer at least partially covering a surface of the base material,
the coating layer comprising a monomolecular layer resulting from surface treatment of the base material with a low-molecular-weight compound having an albumin-binding functional group,
the device satisfying at least one of conditions:
(1) the coating layer includes the monomolecular layer at its uppermost surface with no polymer layer on a surface of the monomolecular layer,
(2) the coating layer has an average thickness of 30 nm or less, and
(3) the coating layer has a surface with at least one of a Si content of 0.5 atm% or more or a P content of 0.5 atm% or more as measured by X-ray photoelectron spectroscopy.

2. A device for use inside a blood vessel, the device comprising: a base material; and a coating layer at least partially covering a surface of the base material,
the coating layer comprising a monomolecular layer resulting from surface treatment of the base material with a low-molecular-weight compound having at least one functional group selected from the group consisting of an amino group, a halogen group, a carboxy group, and an alkyl group,
the device satisfying at least one of conditions:
(1) the coating layer includes the monomolecular layer at its uppermost surface with no polymer layer on a surface of the monomolecular layer,
(2) the coating layer has an average thickness of 30 nm or less, and
(3) the coating layer has a surface with at least one of a Si content of 0.5 atm% or more or a P content of 0.5 atm% or more as measured by X-ray photoelectron spectroscopy.

3. The device according to claim 1 or 2, wherein the device is to be placed inside a blood vessel.

4. The device according to claim 1 or 2, wherein the base material has a linear portion.

5. A coating agent for use in manufacture of the device according to claim 1, the coating agent comprising a low-molecular-weight compound having an albumin-binding functional group.

6. A coating agent for use in manufacture of the device according to claim 2, the coating agent comprising a low-molecular-weight compound having at least one functional group selected from the group consisting of an amino group, a halogen group, a carboxy group, and an alkyl group.
